(19) Europäisches Patentamt
European Patent Office
Office européen des brevets

(11) Veröffentlichungsnummer: **0 361 467 A2**

# (12) EUROPÄISCHE PATENTANMELDUNG

(21) Anmeldenummer: **89117931.9**

(51) Int. Cl.⁵: **C07H 17/04 , A61K 31/70**

(22) Anmeldetag: **28.09.89**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4137 und DSM 3816.

(30) Priorität: **30.09.88 DE 3833180**

(43) Veröffentlichungstag der Anmeldung: **04.04.90 Patentblatt 90/14**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Hammann, Peter, Dr.**
**Mörikestrasse 6**
**D-6233 Kelkheim(Taunus)(DE)**
Erfinder: **Kretzschmar, Gerhard, Dr.**
**Ulmenweg 10**
**D-6236 Eschborn(DE)**
Erfinder: **Schlingmann, Merten, Prof. Dr.**
**Schneidhainer Strasse 32 A**
**D-6240 Königstein(Taunus)(DE)**

(54) **Enolether des Elaiophylins, Verfahren zu deren Herstellung, diese enthaltenden Mittel und ihre Verwendung.**

(57) Verbindung III und IV

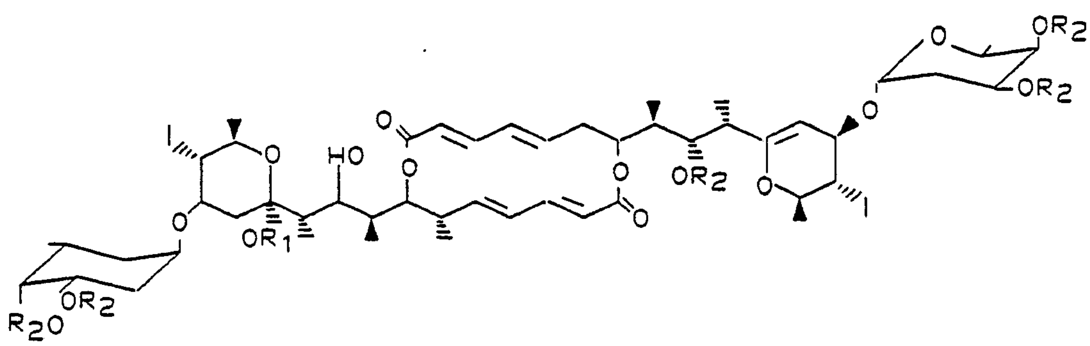

III

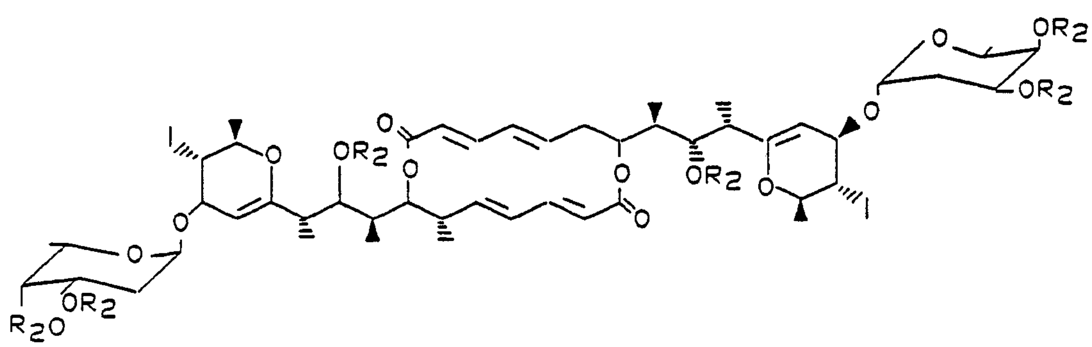

IV

EP 0 361 467 A2

mit (R(1) H, $CH_3$ und R(2) oder Acyl weisen hervorragende anthelminitsche und antibakterielle Wirksamkeit auf. Sie werden aus Diemthylelaiophylin durch ein- bzw. achttägige Reaktion mit dem entsprechenden Säureanhydrid erhalten.

**Enolether des Elaiophylins, Verfahren zu deren Herstellung, diese enthaltenden Mittel und ihre Verwendung.**

Die Erfindung betrifft Enolether des Elaiophylins, Verfahren zu ihrer Herstellung, Arzneimittel aus diesen Enolethern des Elaiophylins und die Verwendung dieser Arzneimittel.

Elaiophylin wurde vor über 25 Jahren zuerst von Arcamone et al. (giorn. Microbiol. 7, 207, 1959) isoliert und später von Arai unter dem Namen Azalomycin B beschrieben. Kaiser et al. (Helv. Chim. Acta, 64 (1981), 407) isolierten Elaiophylin zusammen mit Nigericin und den Niphitricinen A und B aus Kulturen eines Stammes von Streptomyces violaceoniger.

Bekannt sind bereits einige Alkyl-, Alkenyl- und Alkinyl-Derivate sowie Phenyl- und Furylalkyl-Derivate in 11- und 11′-Position am Elaiophylin (Japanische Offenlegungsschrift 61-36295, YOKURA et al.). Ebenfalls bekannt sind die reduzierten und teilreduzierten Halbacetale des Elaiophylins, wobei der 6′-Ring am C11 bzw. am C11 und 11′ geöffnet ist sowie die Tetraacetylderivate (3″,3‴,4″,4‴-Tetra-O-acetyl) des Elaiophylins (s. loc. cit. Kaiser et al.). Sowohl von den reduzierten Halbacetalen als auch von den Tetraacetylderivaten sind schon die Verbindungen bekannt, in denen die C-C-Doppelbindungen des Macrodiolidrings vollständig hydriert sind (s. loc. cit. Kaiser et al.).

Es wurde nun gefunden, daß bei Umsetzung von Dimethylelaiophylin unter Acylierungsbedingungen nach 8 Tagen die entsprechenden C-11-Enolether-Derivate entstehen, die sowohl interessante Zwischenverbindungen für weitere Derivatisierungen als auch gute antibakterielle Wirkstoffe sind. Weiterhin zeigen diese Verbindungen eine gute anthelmintische Aktivität gegen Schafnematoden.

Die Erfindung betrifft daher Verbindungen III

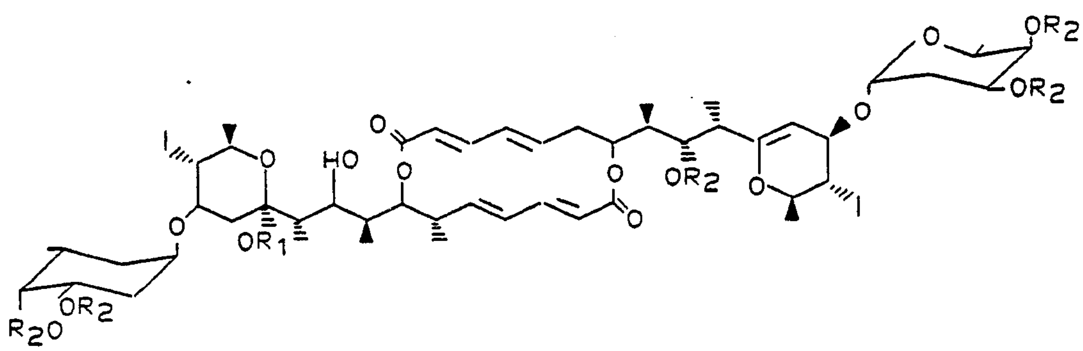

III

und IV

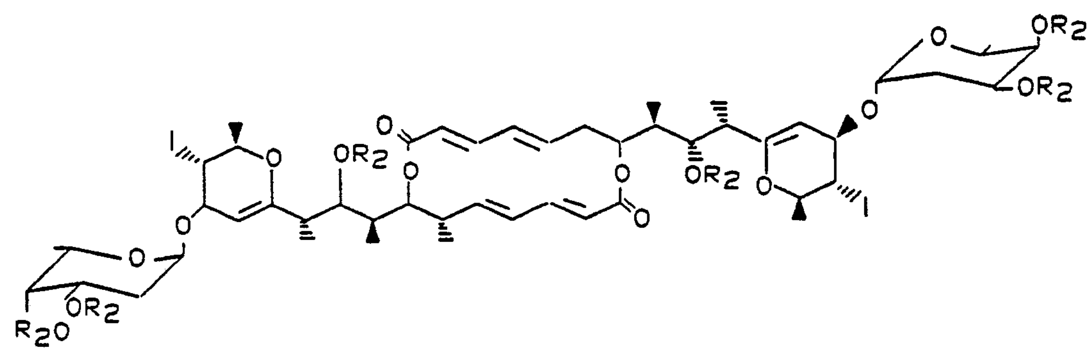

IV

in welchen bedeuten:

R(1) H oder $CH_3$

R(2) Wasserstoff oder einen Rest der Formel

$R(3)-(CH2)n-\underset{\underset{O}{\|}}{C}-$ ,

in welcher

n 0 bis 3 und



F

R(3) ($C_1$ - $C_{15}$)-Alkyl, ($C_2$ - $C_{15}$)-Alkenyl, ($C_2$ - $C_{15}$)-Alkinyl, ($C_3$ -$C_9$)-Cycloalkyl, Phenyl, Naphthyl, Furyl, Thienyl bedeutet, wobei die letzten vier Ringsysteme unsubstituiert oder durch F, Cl, Br, I, nitro, cyano, ($C_1$ - $C_4$)-Alkyl oder ($C_1$ - $C_4$)-Alkoxy substituiert sind.

Insbesondere betrifft die Erfindung Elaiophylinderivate der Formel III oder IV, worin $R^1$ H oder $CH_3$ ist und $R^2$ Wasserstoff oder
einen Rest der Formel V
$R(3)\text{-}(CH_2)_n\ \overset{|}{C}=O$ V
bedeutet, in der
n 0 bis 3 und
$R^3$ $C_1$-$C_5$-Alkyl, $C_2$-$C_5$-Alkenyl, $C_2$-$C_6$-Alkinyl $C_3$-$C_6$-Cycloalkyl, Phenyl oder Furyl oder Thienyl bedeutet, wobei die Phenyl-, Furyl- und Thienylreste unsubstituiert oder durch F, Cl, Br, I, nitro-, cyano-, $C_1$-$C_4$-alkyl- oder $C_1$-$C_4$-alkoxy substituiert sind.

Besonders bevorzugt sind Elaiophylinderivate der Formel III bzw. IV, in welcher
R(1) gleich H oder $CH_3$ ist und
$R^2$ Wasserstoff oder ein Rest der Formel V
$R(3)\text{-}(CH_2)_n\ \overset{|}{C}=O$ V
ist mit
n 1
$R^3$ $C_1$-$C_5$-Alkyl, Cyclohexyl, Phenyl, Furyl oder Thienyl bedeutet, wobei die Phenyl-, Furyl-, und Thienylreste unsubstituiert oder mit Fluor, Chlor oder Brom substituiert sind.

Des weiteren betrifft die Erfindung ein Verfahren zur Herstellung von Elaiophylinderivaten der Formel III und IV, nach Anspruch 1, das dadurch gekennzeichnet ist, daß man
a) Dimethylelaiophylin I

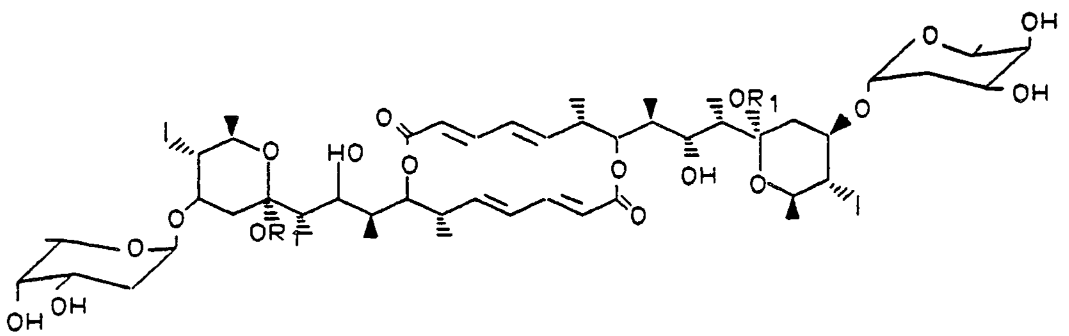

I

mit R(1) = $CH_3$
mit einem Überschuß eines Anhydrides der Formel V
$[R(3)\ (CH_2)_nCO]_2\ O$ V
umsetzt, in der n null bis 3 und
R(3) ($C_1$ -$C_{15}$)-Alkyl, ($C_2$ - $C_{15}$)-Alkenyl, ($C_2$ - $C_{15}$)-Alkinyl, ($C_3$ - $C_9$)-Cycloalkyl, Phenyl, Naphthyl, Furyl, Thienyl bedeuten, wobei die letzten vier Ringsysteme unsubstituiert oder durch F,Cl, Br, I, nitro, cyano, ($C_1$ -$C_4$)-Alkyl oder ($C_1$ - $C_4$)-alkoxy substituiert sind,
zu Verbindungen II, die nach einem Tag entstanden sind,

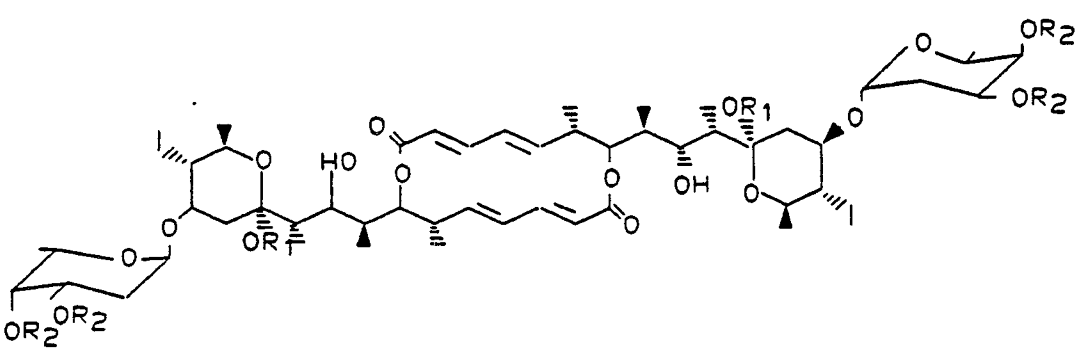

II

in denen
R(2) einen Rest der Formel
$R(3)-(CH_2)_n-CO-$,
bedeutet, in welcher
n 0 bis 3 und
R(3) ($C_1$ -$C_{15}$)-Alkyl, ($C_2$ - $C_{15}$)-Alkenyl, ($C_2$ - $C_{15}$)-Alkinyl, ($C_3$ -$C_9$)-Cycloalkyl, Phenyl, Naphthyl, Furyl, Thienyl bedeuten, wobei die letzten vier Ringsysteme unsubstituiert oder durch F,Cl, Br, I, nitro, cyano, ($C_1$ -$C_4$)-Alkyl oder ($C_1$ - $C_4$)-Alkoxy substituiert sind, bedeutet,
und daß man danach während weiterer 7 Tage zu Verbindungen III mit R(1) = $CH_3$ und R(2) wie oben definiert, oder IV mit R(2) wie oben definiert umsetzt,
oder daß man
b) Elaiophylin I

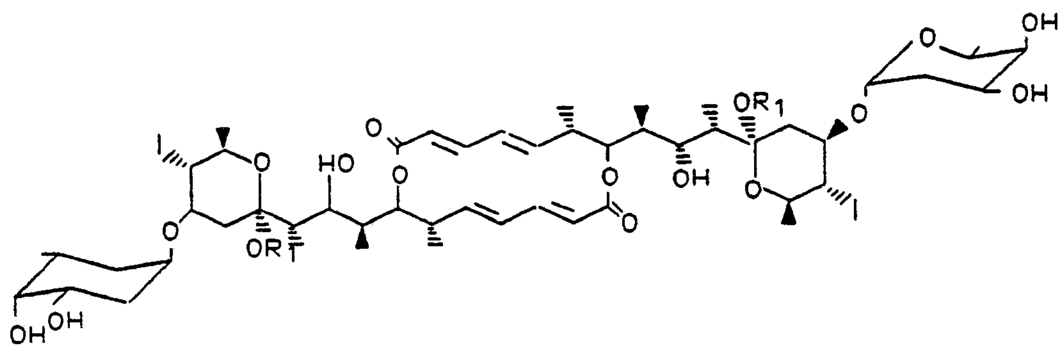

I

mit R(1) = H oder ein Tetraacylderivat II des Elaiophylins

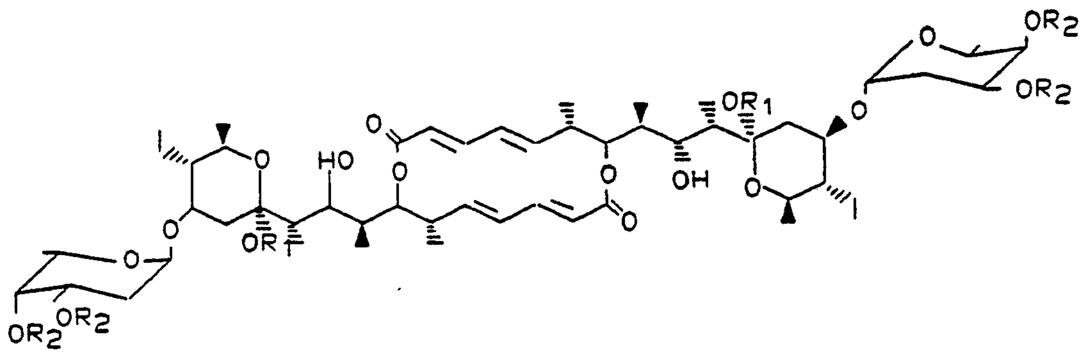

II

mit R(1) = H und R(2), wie unter Variante a) definiert, umsetzt mit einem geringen Überschuß an Trifluoro- oder Trichloroessigsäureanhydrid zu Verbindungen III

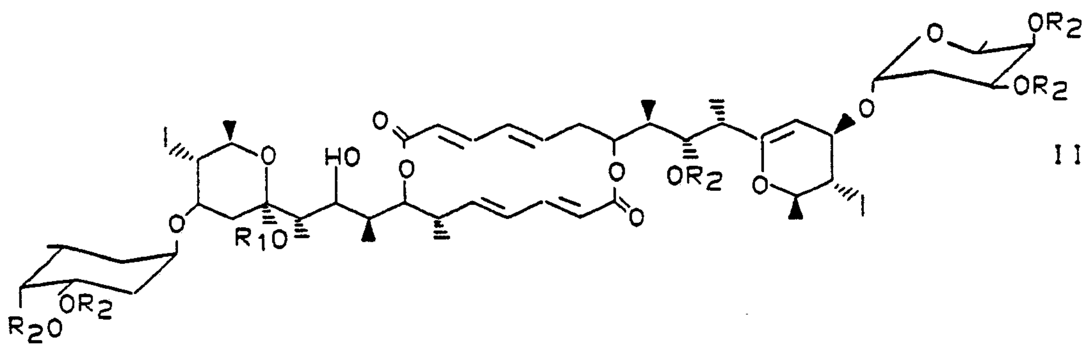

III

mit R(1) = H und
R(2) wie unter Variante a) definiert
oder zu Verbindungen IV

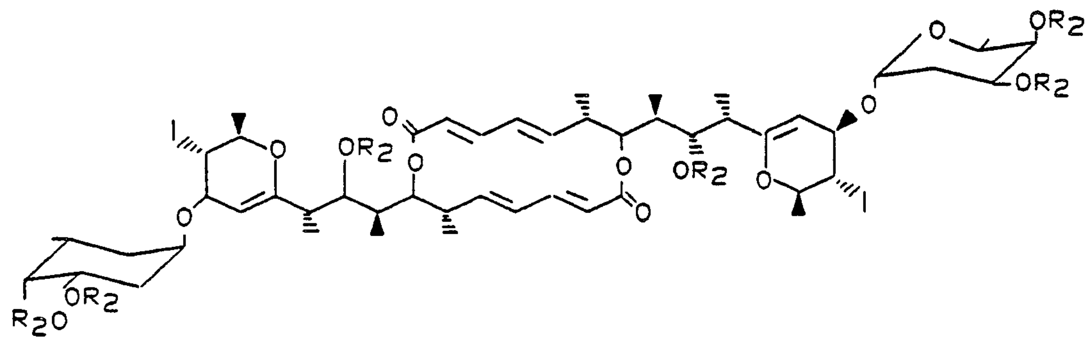

mit R(2), wie unter Variante a) definiert.

Bei der Verfahrensvariante a) geht man am besten so vor, daß man Dimethylelaiophylin mit R(1) = $CH_3$ in äquimolaren Mengen und in bis zu einem 50-fachen Überschuß, gegebenenfalls in einem inerten aprotischen Lösungsmittel, wie Chloroform, Methylenchlorid, Tetrahydrofuran (THF), Essigester oder Dioxan, mit einer Verbindung der Formel V bis zur Beendigung der Reaktion umsetzt, gegebenenfalls in Gegenwart einer Base, vorzugsweise Pyridin.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C. Die Reaktionszeiten betragen 1 bis 600 Stunden, bevorzugt 1 bis 300 Stunden, besonders bevorzugt 1 bis 200 Stunden. Die Beendigung der Reaktion kann beispielsweise mittels Dünnschichtchromatographie bestimmt werden (DC-Kontrolle).

Das als Ausgangssubstanz benötigte Elaiophylin kann beispielsweise nach dem in der deutschen Patentanmeldung P 3721722.4 beschriebenen Verfahren hergestellt werden. Dabei fällt Elaiophylin als Fermentationsprodukt von Kulturen der Stämme Streptomyces violaceoniger DSM 4137 bzw. Streptomyces parvulus DSM 3816 an.

Hieraus wird Dimethylelaiophylin nach dem Verfahren der japanischen Offenlegungsschrift 61-36295 oder nach dem Verfahren der deutschen Patentanmeldung P 3721722.4 (HOE 87/F 321) hergestellt.

Die Ausgangsverbindungen der Formel V sind, sofern nicht käuflich, auf einfache Weise nach literaturbekannten Verfahren herstellbar.

Bei der Verfahrensvariante b) geht man am besten so vor, daß man das Elaiophylin (Formel I, R(1) = H) oder ein Elaiophylin-Derivat (Formel II, R(1) = H, R(2) wie unter a) beschrieben) mit dem 2,2-fachen Überschuß an Trifluoracetanhydrid oder Trichloracetanhydrid, gegebenenfalls in einem inerten aprotischen Lösungsmittel wie Chloroform, Methylenchlorid, Tetrahydrofuran, Essigester oder Dioxan, bis zur Beendigung der Reaktion, gegebenenfalls in Gegenwart einer Base, vorzugsweise Pyridin, umsetzt.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C. Die Reaktionszeiten betragen 1 bis 600 Stunden, bevorzugt 1 bis 300 Stunden, besonders bevorzugt 1 bis 200 Stunden. Die Beendigung der Reaktion kann beispielsweise mittels Dünnschichtchromatographie bestimmt werden (DC-Kontrolle).

Die benötigten Acyl-Derivate mit R(1) = H und R(2), wie unter Verfahrensvariante a) beschrieben, werden nach dem Verfahren der deutschen Patentanmeldung P 3721722.4 (HOE 87/F 321), in Analogie zum unter Verfahrensvariante a) beschriebenen Verfahren, jedoch mit den vorstehend angegebenen Reaktionszeiten hergestellt.

Die Reiniigung, Isolierung und Aufarbeitung der Substanzen erfolgt nach den üblichen Methoden; beispielsweise können die Reaktionsprodukte durch Chromatographie an polaren Trägermaterialien wie Kieselgel oder ®Sephadex LH 20 mit Lösungsmitteln wie niederen Alkanolen wie Methanol oder Chloroform oder Essigester oder Methanol/Chloroform-Mischungen, aber auch durch extraktive Methoden, wie flüssig/flüssig-Extraktion oder fest/flüssig-Extraktion oder durch Kristallisation, gereinigt werden.

Die Derivate des Elaiophylins zeigen anthelmintische Wirkung, insbesondere gegen Haemonchus, Trichostrongylus, Ostertagia, Cooperia, Chabertia, Strongyloides, Oesophagostomum, Hyostrongylus, Ancylostoma, Ascaris und Heterakis. Besonders ausgeprägt ist die Wirksamkeit gegenüber Magen-Darm-Strongyliden und Lungenwürmern, von denen vor allem Haus- und Nutztiere befallen werden. Sie zeigen ebenfalls ausgeprägte antibakterielle Wirkung.

Die Elaiophylinderivate können grundsätzlich als solche in Substanz verabreicht werden. Bevorzugt ist die Verwendung in Mischung mit geeignetem Trägermaterial. Als Trägermaterial können die üblichen

Futtermittelmischungen verwendet werden.

In den sich anschließenden Beispielen wird die Erfindung weiter erläutert. Prozentangaben beziehen sich auf das Gewicht und Mischungsverhältnisse bei Flüssigkeiten beziehen sich auf das Volumen, wenn keine anderen Angaben gemacht wurden.

**Verfahren 1**

1 mmol Elaiophylin werden in 10 ml Methanol gelöst und mit 70 mg $FeCl_3$ bei Zimmertemperatur gerührt. Nach der Zugabe von 100 ml Ethylacetat wird zweimal mit 50 ml einer gesättigten EDTA-Lösung (pH = 7, mit NaOH einstellen) und zweimal mit 50 ml Wasser gewaschen. Nach dem Trocknen der organischen Phase über Natriumsulfat wird Vakuum bis zu einem festen Rückstand eingeengt.

**Verfahren 2**

1 mmol Dimethylelaiophylin wird in 20 ml Pyridin mit 4 ml Acetanhydrid bei Zimmertemperatur für 8 d gerührt. Nach der Hydrolyse mit Wasser (60 ml) wird mit Essigester extrahiert. Die organische Phase wird mit 0,1 N HCL und Wasser gewaschen, über Natriumsulfat getrocknet und im Vakuum eingeengt. Der verbleibende Rückstand wird an Kieselgel chromatographiert (Elutionsmittel Essigester/Hexan 1:3 auf 1:1)

**Verfahren 3**

1 mmol Elaiophylin wird in 20 ml Pyridin mit 2,2 mmol Trifluoressigsäureanhydrid für 12 h bei Zimmertemperatur gerührt. Die Aufarbeitung erfolgt analog Verfahren 2. Der verbleibende Rückstand wird an Kieselgel chromatographiert (Elutionsmittel $CH_2Cl_2/CH_3OH$ 20:1)

**Beispiel 1**

**Tabelle 1**

| | Formel | $R_1$ | $R_2$ | Synthese aus | Reagens | Reaktionszeit | Verfahren | Ausb. |
|---|---|---|---|---|---|---|---|---|
| 1 | I | $CH_3$ | H | Elaiophylin | $FeCl_3$/MeOH | 5 min. | 1 | 100 % |
| 2 | III | $CH_3$ | $CH_3CO$ | 1 | $(CH_3CO)_2O$/ Pyridin | 8 Tage | 2 | 37 % |
| 3 | IV - | | $CH_3CO$ | | | | | 32 % |
| 4 | III | H | H | Elaiophylin | $(CF_3CO)_2O$/ Pyridin | 12 h | 3 | 24 % |
| 5 | IV - | | H | | | | | 52 % * |

* Nach 10 Tagen entstehen 82% Verbindung 5

Tabelle 2

| Physiko-chemische Daten | | | |
|---|---|---|---|
| | M | MNa⁺ | NMR $^{13}C$ in $CDCl_3$ |
| 1 | 1053.35 | | |
| 2 | 1231.5 | 1253 | |
| 3 | 1241.5 | 1263 | 170.8, 170.3, 170.1, 167.4, 156.7 195.4, 144.4, 131.7, 122.2, 96.3, 94.9 |
| 4 | 1007.3 | 1028 | |
| 5 | 989.3 | 1011 | 167.4, 155.0, 145.5, 144.0, 131.9, 122.0, 98.0, 93.5 |

## Ansprüche

1. Verbindung III und IV

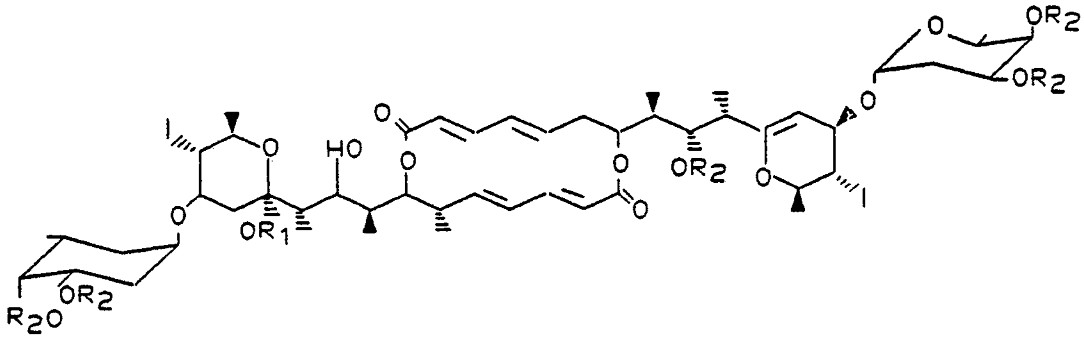

III

und IV

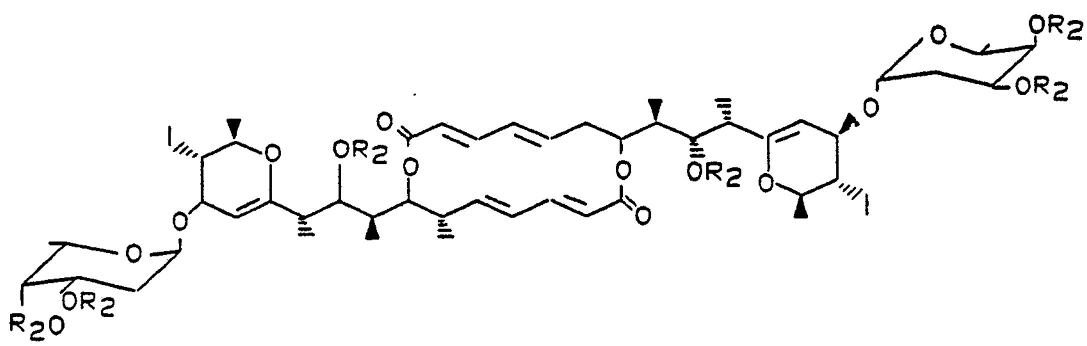

IV

in welchen bedeuten:
R(1) H oder $CH_3$
R(2) Wasserstoff oder einen Rest der Formel
$R(3)\text{-}(CH2)n\text{-}\underset{\underset{O}{\|}}{C}\text{-}$ ,
in welcher
n 0 bis 3 und
R(3) ($C_1$ - $C_{15}$)-Alkyl, ($C_2$ - $C_{15}$)-Alkenyl, ($C_2$ - $C_{15}$)-Alkinyl, ($C_3$ -$C_9$)-Cycloalkyl, Phenyl, Naphthyl, Furyl, Thienyl bedeutet, wobei die letzten vier Ringsysteme unsubstituiert oder durch F, Cl, Br, I, nitro, cyano, ($C_1$ - $C_4$)-Alkyl oder ($C_1$ - $C_4$)-Alkoxy substituiert sind.

2. Verbindung III oder IV nach Anspruch 1, dadurch gekennzeichnet, daß
R(1) H oder $CH_3$ und
R(2) Wasserstoff oder einen Rest der Formel V

$R(3)-(CH_2)_n-\overset{|}{C}=O$ V

bedeutet, in der

n 1 bis 3 und

$R^3$ $C_1$-$C_5$-Alkyl, $C_2$-$C_5$-Alkenyl, $C_2$-$C_6$-Alkinyl, $C_3$-$C_6$-Cycloalkyl, Phenyl oder Furyl oder Thienyl bedeutet, wobei die Phenyl-, Furyl-und Thienylreste unsubstituiert oder durch F, Cl, Br, I, nitro-, cyano-, $C_1$-$C_4$-alkyl-oder $C_1$-$C_4$-alkoxy substituiert sind.

3. Verbindung III oder IV nach Anspruch 1, dadurch gekennzeichnet, daß

R(1) gleich H oder $CH_3$ und

$R^2$ Wasserstoff oder einen Rest der Formel V

$R(3)-(CH_2)_n-\overset{|}{C}=O$ V

n 1

$R^3$ $C_1$-$C_5$-Alkyl, Cyclohexyl, Phenyl, Furyl oder Thienyl bedeutet, wobei die Phenyl-, Furyl-, und Thienylreste gegebenenfalls mit Fluor, Chlor oder Brom substituiert sind.

4. Verfahren zum Herstellen einer Verbindung III oder IV nach Anspruch 1, dadurch gekennzeichnet, daß man

a) Dimethylelaiophylin I

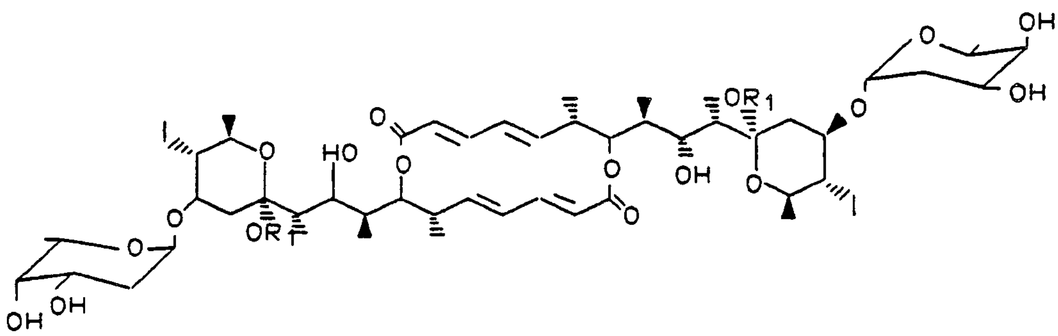

I

mit R(1) = $CH_3$

mit einem Überschuß eines Anhydrides der Formel V

$[R(3)\ (CH_2)_nCO]_2\ O$ V

umsetzt, in der n null bis 3 und

R(3) ($C_1$ -$C_{15}$)-Alkyl, ($C_2$ - $C_{15}$)-Alkenyl, ($C_2$ - $C_{15}$)-Alkinyl, ($C_3$ - $C_9$)-Cycloalkyl, Phenyl, Naphthyl, Furyl, Thienyl bedeuten, wobei die letzten vier Ringsysteme unsubstituiert oder durch F,Cl, Br, I, nitro, cyano, ($C_1$ -$C_4$)-Alkyl oder ($C_1$ - $C_4$)-alkoxy substituiert sind,

zu Verbindungen II, die nach einem Tag entstanden sind,

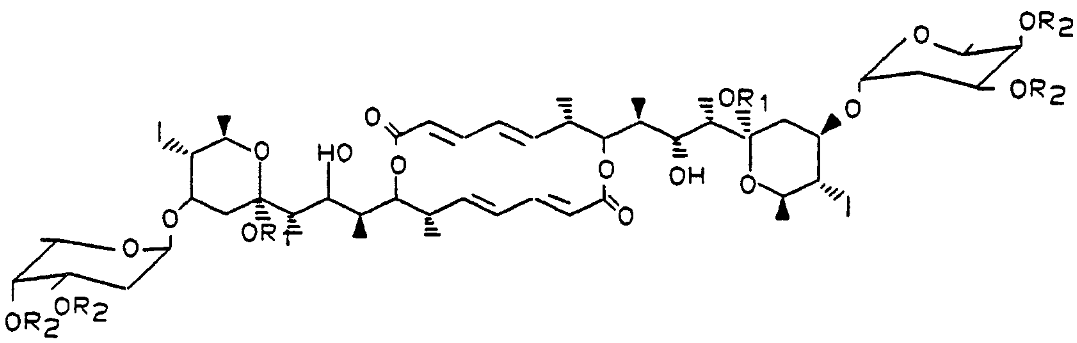

II

in denen R(2) einen Rest der Formel

$R(3)-(CH_2)_n-CO-$,

bedeutet, in welcher

n 0 bis 3 und

R(3) ($C_1$ -$C_{15}$)-Alkyl, ($C_2$ - $C_{15}$)-Alkenyl, ($C_2$ - $C_{15}$)-Alkinyl, ($C_3$ -$C_9$)-Cycloalkyl, Phenyl, Naphthyl, Furyl, Thienyl bedeuten, wobei die letzten vier Ringsysteme unsubstituiert oder durch F,Cl, Br, I, nitro, cyano, ($C_1$

$-C_4)$-Alkyl oder $(C_1 - C_4)$-Alkoxy substituiert sind, bedeutet,
und daß man danach während weiterer 7 Tage zu Verbindungen III mit R(1) = $CH_3$ und R(2) wie oben definiert, umsetzt, oder daß man
b) Elaiophylin I

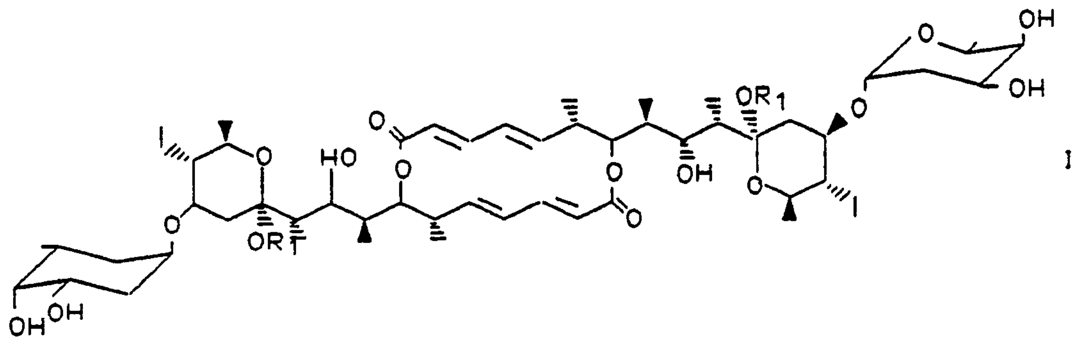

I

mit R(1) = H oder ein Tetraacylderivat II des Elaiophylins mit R(1) = H und R(2), wie unter Variante a) definiert, umsetzt mit einem geringen Überschuß an Trifluoro- oder Trichloroessigsäureanhydrid zu Verbindungen III

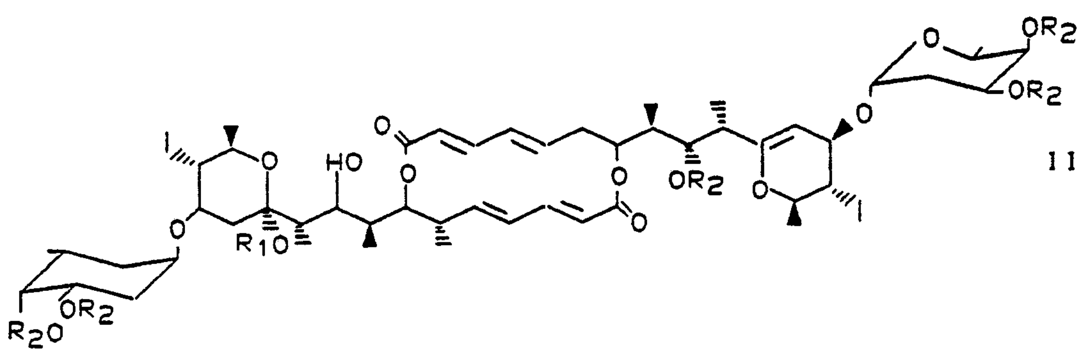

III

mit R(1) = H und
R(2) wie unter Variante a) definiert
oder zu Verbindungen IV

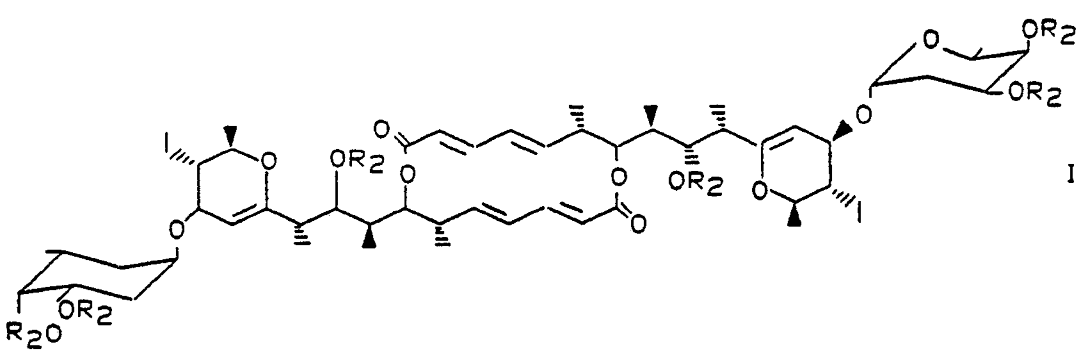

IV

mit R(2), wie unter Variante a) definiert.

5. Anthelmintikum, gekennzeichent durch einen wirksamen Gehalt einer Verbindung III oder IV nach Anspruch 1.

6. Verwendung einer Verbindung III oder IV nach Anspruch 1 als Anthelmintikum.

7. Verwendung einer Verbindung I nach Anspruch 1 zur Herstellung eines Anthelmintikums.

8. Verwendung einer Verbindung I nach 1 zur Herstellung eines antibakteriellen Medikaments.